# EUROPÄISCHE PATENTSCHRIFT

(11) **EP 0 821 580 B1**
(45) Veröffentlichungstag und Bekanntmachung des Hinweises auf die Patenterteilung: **01.08.2001**
(21) Anmeldenummer: 96910962.8
(22) Anmeldetag: 12.04.1996
(51) Int. Cl.: A61K 7/50

(54) **HAARBEHANDLUNGSMITTEL**
HAIR-TREATMENT AGENTS
AGENTS DE SOINS CAPILLAIRES

(30) Priorität: 20.04.1995 DE 19514557
(43) Veröffentlichungstag der Anmeldung: 04.02.1998
(73) Patentinhaber: Cognis Deutschland GmbH, 40589 Düsseldorf (DE)
(72) Erfinder: FABRY, Bernd, D-41352 Korschenbroich (DE)
(86) Internationale Anmeldenummer: EP9601555
(87) Internationale Veröffentlichungsnummer: WO9632928

(56) Entgegenhaltungen:
- EP-A- 0 367 939
- EP-A- 0 571 086
- EP-A- 0 638 639
- DE-A- 1 950 643
- DE-A- 3 527 974
- US-A- 4 370 272
- US-A- 5 429 755

## Beschreibung

Die Erfindung betrifft die Verwendung von quartären Glycerinstickstoffverbindungen zur Herstellung von Haarbehandlungsmitteln.

### Stand der Technik

Der Einsatz insbesondere von Stickstofftensiden mit Esterstruktur, sogenannter Esterquats, im Bereich der Textil- und Haaravivage sowie in kosmetischen Produkten ist bekannt. Übersichten hierzu finden sich beispielsweise von O.Ponsati in **C.R. 23.CED-Kongress, Barcelona, S.167 (1992)** und R.Puchta in C.R. **24.CED-Kongress, Sitges, S.59 (1993).** Stellvertretend für den umfangreichen druckschriftlichen Stand der Technik sei auf die Deutschen Patentanmeldungen **DE-A1 4138630, DE-A1 4305726** und **DE-A 4309567** (Henkel) sowie die Europäischen Patentanmeldungen **EP-A 0367939** (Wella), **EP-A1 0507003** (Rewo) und **EP-A1 0636356** (Stepan) verwiesen. Die in den genannten Druckschriften offenbarten Kationtenside sind jedoch im Hinblick auf ihre Verwendung in Haarbehandlungsmitteln bezüglich Weichgriff, Verminderung der elektrostatischen Aufladung, Kaltwasserdispergierbarkeit, Viskosität und Lagerstabilität nicht immer voll befriedigend.

Aus der **DE-OS 1950643** sind Kondensationsprodukte von Myristyloxypropyldiethanolamin und Glycerinchlorpropionsäureester und deren Verwendung als Haarbehandlungsmittel bekannt. Gegenstand der **DE-A1 3527974** sind saure Haarpflegemittel mit einem Gehalt an Betainestern.

Demzufolge hat die Aufgabe der Erfindung nun darin bestanden, neue Haarbehandlungsmittel mit einem Gehalt an kationischen Tensiden zur Verfügung zu stellen, die frei von den geschilderten Nachteilen sind.

Gegenstand der Erfindung ist die Verwendung von quartären Glycerinstickstoffverbindungen der Formel **(I)**, 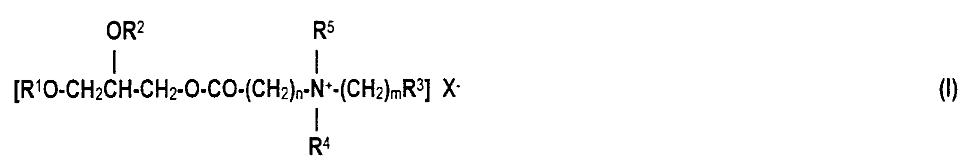 in der R¹ und R² unabhängig voneinander für Wasserstoff oder einen linearen oder verzweigten, gesättigten Acylrest mit 12 bis 22 Kohlenstoffatomen, R³ für eine Methylgruppe oder einen linearen oder verzweigten, gesättigten Oxyacylrest mit 12 bis 22 Kohlenstoffatomen, R⁴ und R⁵ unabhängig voneinander für einen gegebenenfalls hydroxysubstituierten Alkylrest mit 1 bis 4 Kohlenstoffatomen, n für Zahlen von 1 bis 3, m für 0 oder Zahlen von 1 bis 18 und X für Halogen oder Alkylsulfat steht, zur Herstellung von Haarbehandlungsmitteln.

Überraschenderweise wurde gefunden, daß Haarbehandlungsmittel, die als kationische Tenside quartäre Glycerinstickstoffverbindungen der Formel **(I)** enthalten, gegenüber Produkten des Stands der Technik verbesserte avivierende und antistatische Eigenschaften sowie eine leichtere Dispergierbarkeit insbesondere in kaltem Wasser aufweisen. Diese Eigenschaften können in Abmischung mit weiteren Tensiden in synergistischer Weise noch verbessert werden.

### Quartäre Glycerinstickstoffverbindungen

Die erfindungsgemäßen quartären Glycerinstickstoffverbindungen stellen bekannte Stoffe dar, die nach den einschlägigen Methoden der präparativen organischen Chemie erhalten werden können. Gemäß der Europäischen Patentanmeldung **EP-A 0638639** (Akzo) kann man zu ihrer Herstellung Glycerin oder technische Monoglyceride zunächst mit Natriumchloracetat verestern, den halogenierten Ester mit einem Fettsäureaminoester kondensieren und diesen dann anschließend quaternieren. Quartäre Glycerinstickstoffverbindungen, die aus anwendungstechnischer Sicht besonders bevorzugt sind, folgen der Formel (I) in der R¹ für Wasserstoff oder einen C₁₂-C₁₈-Kokosacylrest, R² für Wasserstoff, R³ für einen C₁₂-C₁₈-Kokosoxyacylrest, R⁴ und R⁵ für Methylgruppen, n für 1, m für 2 und X für Chlorid oder Methylsulfat steht.

### Tenside

Die erfindungsgemäßen Haarbehandlungsmittel können weitere kationische, nichtionische und/oder amphotere bzw. zwitterionische Tenside enthalten. Typische Beispiele für **kationische Tenside** sind quartäre Ammoniumverbindungen und Esterquats, insbesondere quatemierte Fettsäuretrialkanolaminester-Salze. Typische Beispiele für **nichtionische Tenside** sind Fettalkoholpolyglycolether, Alkylphenolpolyglycolether, Fettsäurepolyglycolester, Fettsäureamidpolygylcolether, Fettaminpolyglycolether, alkoxylierte Triglyceride, Mischether bzw. Mischformale, Alk(en)yloligoglykoside, Fettsäure-N-alkylglucamide, Proteinhydrolysate (insbesondere pflanzliche Produkte auf Sojabasis), Polyolfettsäureester, Zuckerester, Sorbitanester und Polysorbate. Sofern die nichtionischen Tenside Polyglycoletherketten enthalten, können sie eine konventionelle, vorzugsweise jedoch eine eingeengte Homologenverteilung aufweisen. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S.54-124 oder J.Falbe (ed.), "Katalysatoren, Tenside und Mineralöladditive", Thieme Verlag, Stuttgart, 1978, S.123-217** verwiesen. In einer bevorzugten Ausführungsform der Erfindung werden die quartären Glycerinstickstoffverbindungen mit Tensiden abgemischt, die ausgewählt sind aus der Gruppe, die von Esterquats, Alkyloligoglucosiden, Fettsäure-N-alkylglucamiden und Betainen gebildet wird. In diesen Fällen wird eine starke synergistische Verstärkung der avivierenden und antistatischen Eigenschaften beobachtet. Die Mittel können die quartären Glycerinstickstoffverbindungen der Formel (I) und die weiteren Tenside im Gewichtsverhältnis 10:90 bis 90:10 enthalten.

### Esterguats

Unter der Bezeichnung Esterquats werden im allgemeinen quaternierte Fettsäuretriethanolaminestersalze verstanden. Es handelt sich dabei um bekannte Stoffe, die man nach den einschlägigen Methoden der präparativen organischen Chemie erhalten kann. In diesem Zusammenhang sei auf die Internationale Patentanmeldung **WO 91/01295** (Henkel) verwiesen, nach der man Triethanolamin in Gegenwart von unterphosphoriger Säure mit Fettsäuren partiell verestert, Luft durchleitet und anschließend mit Dimethylsulfat oder Ethylenoxid quaterniert. Stellvertretend für den umfangreichen Stand der Technik sei an dieser Stelle auf die Druckschriften **US 3915867, US 4370272, EP-A1 0239910, EP-A1 0293955, EP-A1 0295739** und **EP-A1 0309052** verwiesen. Die quaternierten Fettsäuretriethanolaminestersalze folgen der Formel **(II)**, 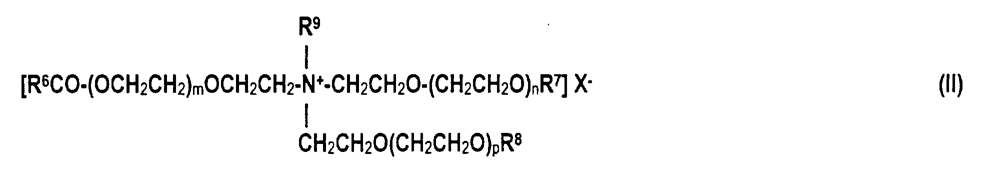 in der R⁶CO für einen Acylrest mit 6 bis 22 Kohlenstoffatomen, R⁷ und R⁸ unabhängig voneinander für Wasserstoff oder R⁶CO, R⁹ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht. Typische Beispiele für Esterquats, die im Sinne der Erfindung Verwendung finden können, sind Produkte auf Basis von Capronsäure, Caprylsäure, Caprinsäure, Laurinsäure, Myristinsäure, Palmitinsäure, Isostearinsäure, Stearinsäure, Ölsäure, Elaidinsäure, Arachinsäure, Behensäure und Erucasäure sowie deren technische Mischungen, wie sie beispielsweise bei der Druckspaltung natürlicher Fette und Öle anfallen. Vorzugsweise werden technische C_{12/18}-Kokosfettsäuren und insbesondere teilgehärtete C_{16/18}-Talg- bzw. Palmfettsäuren sowie elaidinsäurereiche C_{16/18}-Fettsäureschnitte eingesetzt. Zur Herstellung der quatemierten Ester können die Fettsäuren und das Triethanolamin im molaren Verhältnis von 1,1 : 1 bis 3 : 1 eingesetzt werden. Im Hinblick auf die anwendungstechnischen Eigenschaften der Esterquats hat sich ein Einsatzverhältnis von 1,2: 1 bis 2,2:1, vorzugsweise 1,5:1 bis 1,9:1 als besonders vorteilhaft erwiesen. Die bevorzugten Esterquats stellen technische Mischungen von Mono-, Di- und Triestern mit einem durchschnittlichen Veresterungsgrad von 1,5 bis 1,9 dar und leiten sich von technischer C_{16/18}-Talg- bzw. Palmfettsäure (lodzahl 0 bis 40) ab. Aus anwendungstechnischer Sicht haben sich quaternierte Fettsäuretriethanolaminestersalze der Formel **(II)** als besonders vorteilhaft erwiesen, in der R¹CO für einen Acylrest mit 16 bis 18 Kohlenstoffatomen, R⁷ für R⁶CO, R⁸ für Wasserstoff, R⁹ für eine Methylgruppe, m, n und p für 0 und X für Methylsulfat steht.

Neben den quaternierten Fettsäuretriethanolaminestersalzen kommen als Esterquats ferner auch quaternierte Estersalze von Fettsäuren mit Diethanolalkylaminen der Formel **(III)** in Betracht, 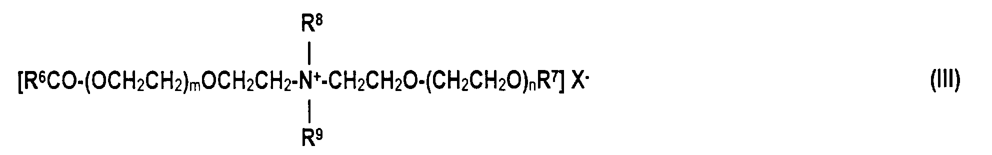 in der R⁶CO für einen Acylrest mit 6 bis 22 Kohlenstoffatomen, R⁷ für Wasserstoff oder R⁶CO, R⁸ und R⁹ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht. Als weitere Gruppe geeigneter Esterquats sind schließlich die quaternierten Estersalze von Fettsäuren mit 1,2-Dihydroxypropyldialkylaminen der Formel **(IV)** zu nennen, 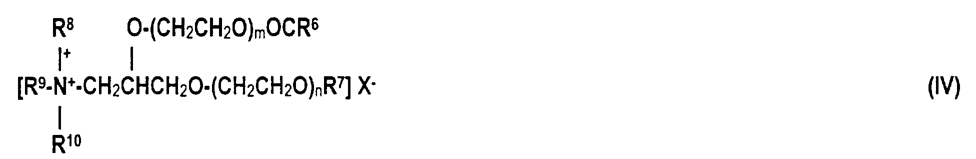 in der R⁶CO für einen Acylrest mit 6 bis 22 Kohlenstoffatomen, R⁷ für Wasserstoff oder R⁶CO, R⁸, R⁹ und R¹⁰ unabhängig voneinander für Alkylreste mit 1 bis 4 Kohlenstoffatomen, x und y in Summe für 0 oder Zahlen von 1 bis 12 und X für Halogenid, Alkylsulfat oder Alkylphosphat steht. Hinsichtlich der Auswahl der bevorzugten Fettsäuren und des optimalen Veresterungsgrades gelten die für **(II)** genannten Beispiele auch für die Esterquats der Formeln **(III)** und **(IV)**. Üblicherweise gelangen die Esterquats in Form 50 bis 90 Gew.-%iger alkoholischer Lösungen in den Handel, die bei Bedarf problemlos mit Wasser verdünnt werden können. Das optimale Einsatzverhältnis im Hinblick auf eine besonders vorteilhafte synergistische Verstärkung der anwendungstechnischen Eigenschaften liegt bei einem Gewichtsverhältnis von **(I)** zu Esterquat von 30:70 bis 50:50.

### Alkyl- und/oder Alkenyloligoglykoside

Alkyl- und Alkenyloligoglykoside stellen bekannte Stoffe dar, die der Formel **(V)** folgen,

**R**^{**11**}**O-[G]**_{**d**} **(V)**

in der R¹¹ für einen Alkyl- und/oder Alkenylrest mit 4 bis 22 Kohlenstoffatomen, G für einen Zuckerrest mit 5 oder 6 Kohlenstoffatomen und d für Zahlen von 1 bis 10 steht, und die nach den einschlägigen Verfahren der präparativen organischen Chemie erhalten werden können. Stellvertretend für das umfangreiche Schrifttum sei hier auf die Schriften **EP-A 0301298** und **WO 90/03977** verwiesen. Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligo**glucoside**. Die Indexzahl d in der allgemeinen Formel (V) gibt den Oligomerisierungsgrad (DP), d. h. die Verteilung von Mono- und Oligoglykosiden an und steht für eine Zahl zwischen 1 und 10. Während d in einer gegebenen Verbindung stets ganzzahlig sein muß und hier vor allem die Werte d = 1 bis 6 annehmen kann, ist der Wert d für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad d von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R¹¹ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese anfallen. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C12-Alkohol verunreinigt sein können sowie Alkyloligoglucoside auf Basis technischer C_{9/11}-Oxoalkohole (DP = 1 bis 3). Der Alkyl- bzw. Alkenylrest R¹¹ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3. Das optimale Einsatzverhältnis im Hinblick auf eine besonders vorteilhafte synergistische Verstärkung der anwendungstechnischen Eigenschaften liegt bei einem Gewichtsverhältnis von **(I)** zu Alkyloligoglucosid von 70:30 bis 40 : 60.

### Fettsäure-N-alkylpolyhydroxyalkylamide

Fettsäure-N-alkylpolyhydroxyalkylamide der Formel **(VI)**, 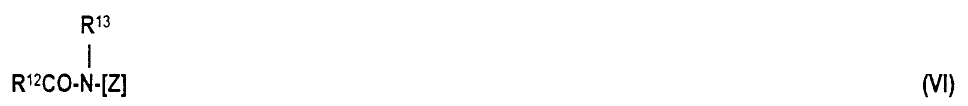 in der R¹²CO für einen aliphatischen Acylrest mit 6 bis 22 Kohlenstoffatomen, R¹³ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht. Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Hinsichtlich der Verfahren zu ihrer Herstellung sei auf die US-Patentschriften **US 1985424, US 2016962** und **US 2703798** sowie die Internationale Patentanmeldung **WO 92/06984** verwiesen. Eine Übersicht zu diesem Thema von H.Kelkenberg findet sich in **Tens. Surf.Det. 25, 8 (1988).** Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher **Fettsäure-N-alkylglucamide** dar, wie sie durch die Formel **(VII)** wiedergegeben werden:

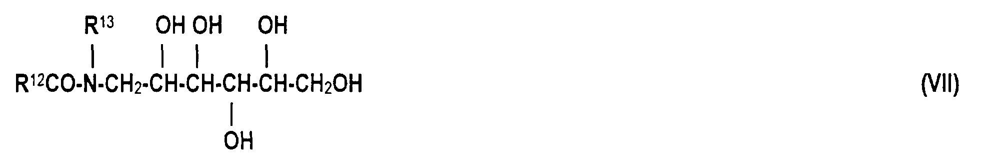

Vorzugsweise werden als Fettsäure-N-alkylpolyhydroxyalkylamide Glucamide der Formel (VII) eingesetzt, in der R¹³ für Wasserstoff oder eine Alkylgruppe steht und R¹²CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkylglucamide der Formel **(VII)**, die durch reduktive Aminierung von Glucose mit Methylamin und anschließende Acylierung mit Laurinsäure oder C_{12/14}-Kokosfettsäure bzw. einem entsprechenden Derivat erhalten werden. Weiterhin können sich die Polyhydroxyalkylamide auch von Maltose und Palatinose ableiten. Das optimale Einsatzverhältnis im Hinblick auf eine besonders vorteilhafte synergistische Verstärkung der anwendungstechnischen Eigenschaften liegt bei einem Gewichtsverhältnis von **(I)** zu Fettsäure-N-alkylglucamid von 70: 30 bis 40: 60.

### Betaine

Betaine stellen bekannte Tenside dar, die überwiegend durch Carboxyalkylierung, vorzugsweise Carboxymethylierung von aminischen Verbindungen hergestellt werden. Vorzugsweise werden die Ausgangsstoffe mit Halogencarbonsäuren oder deren Salzen, insbesondere mit Natriumchloracetat kondensiert, wobei pro Mol Betain ein Mol Salz gebildet wird. Ferner ist auch die Anlagerung von ungesättigten Carbonsäuren wie beispielsweise Acrylsäure möglich. Zur Nomenklatur und insbesondere zur Unterscheidung zwischen Betainen und "echten" Amphotensiden sei auf den Beitrag von U.Ploog in **Seifen-Öle-Fette-Wachse, 198, 373 (1982)** verwiesen. Weitere Übersichten zu diesem Thema finden sich beispielsweise von A.O'Lennick et al. in **HAPPI, Nov. 70 (1986),** S.Holzman et al. in **Tens.Det. 23, 309 (1986),** R.Bibo et al. in **Soap Cosm.Chem.Spec. Apr. 46 (1990)** und P.Ellis et al. in **Euro Cosm. 1, 14 (1994).** Beispiele für geeignete Betaine stellen die Carboxyalkylierungsprodukte von sekundären und insbesondere tertiären Aminen dar, die der Formel **(VIII)** folgen, 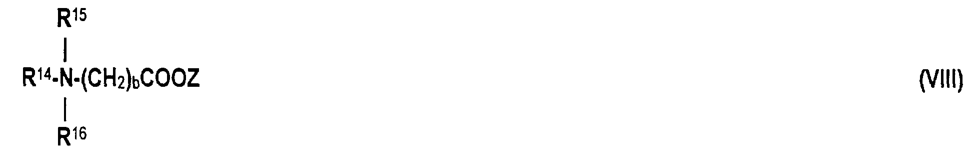 in der R¹⁴ für Alkyl- und/oder Alkenylreste mit 6 bis 22 Kohlenstoffatomen, R¹⁵ für Wasserstoff oder Alkylreste mit 1 bis 4 Kohlenstoffatomen, R¹⁶ für Alkylreste mit 1 bis 4 Kohlenstoffatomen, b für Zahlen von 1 bis 3 und Z für ein Alkali- und/oder Erdalkalimetall oder Ammonium steht. Typische Beispiele sind die Carboxymethylierungsprodukte von Hexylmethylamin, Hexyldimethylamin, Octyldimethylamin, Decyldimethylamin, Dodecylmethylamin, Dodecyldimethylamin, Dodecylethylmethylamin, C_{12/14}-Kokosalkyldimethylamin, Myristyldimethylamin, Cetyldimethylamin, Stearyldimethylamin, Stearylethylmethylamin, Oleyldimethylamin, C_{16/18}-Talgalkyldimethylamin sowie deren technische Gemische.

Weiterhin kommen auch Carboxyalkylierungsprodukte von **Amidoaminen** in Betracht, die der Formel **(IX)** folgen, 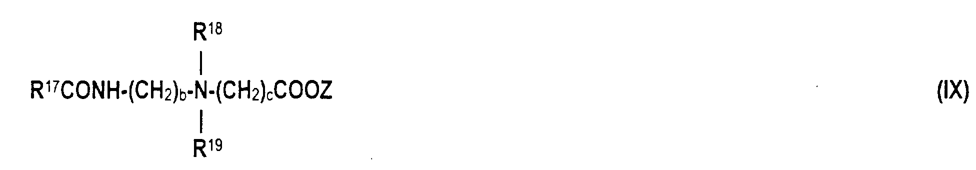 in der R¹⁷CO für einen aliphatischen Acylrest mit 6 bis 22 Kohlenstoffatomen und 0 oder 1 bis 3 Doppelbindungen, R¹⁸ für Wasserstoff oder Alkylreste mit 1 bis 4 Kohlenstoffatomen, R¹⁹ für Alkylreste mit 1 bis 4 Kohlenstoffatomen, b für Zahlen von 1 bis 3, c für Zahlen von 1 bis 6 und Z für ein Alkaliund/oder Erdalkalimetall oder Ammonium steht. Typische Beispiele sind Umsetzungsprodukte von Fettsäuren mit 6 bis 22 Kohlenstoffatomen, namentlich Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Gemische, mit N,N-Dimethylaminoethylamin, N,N-Dimethylaminopropylamin, N,N-Diethylaminoethylamin und N,N-Diethylaminopropylamin, die mit Natriumchloracetat kondensiert werden. Bevorzugt ist der Einsatz eines Kondensationsproduktes von C_{8/18}-Kokosfettsäure-N,N-dimethylaminopropylamid mit Natriumchloracetat. Das optimale Einsatzverhältnis im Hinblick auf eine besonders vorteilhafte synergistische Verstärkung der anwendungstechnischen Eigenschaften liegt bei einem Gewichtsverhältnis von **(I)** zu Betain von 80 : 20 bis 90:10.

### Herstellverfahren

Ein besonderer Vorteil der erfindungsgemäß einzusetzenden quartären Glycerinstickstoffverbindungen besteht darin, daß sie infolge der Glycerinesterbindung über eine besonders vorteilhafte biologische Abbaubarkeit verfügen. Umgekehrt bedeutet dies aber, daß sie als reine wäßrig verdünnte Lösung, zumal unter den sauren Bedingungen üblicher Haarbehandlungsmittel, nicht unbegrenzt lange lagerstabil sind. Vorzugsweise werden die quartären Glycerinstickstoffverbindungen der Formel **(I)** hergestellt, indem man Glycerin oder Glycerinpartialester mit Halogencarbonsäuren - oder deren Salzen verestert, die resultierenden halogenierten Ester mit sekundären Aminverbindungen kondensiert und anschließend in Gegenwart von Dispergatoren bzw. Emulgatoren in an sich bekannter Weise quaterniert. Typische Beispiele für geeignete Halogencarbonsäuren bzw. entsprechende Derivate sind Chloressigsäure bzw. deren Alkalisalze. Für die Kondensationsreaktion kommen als sekundäre Aminverbindungen beispielsweise Methylstearylamin oder insbesondere Fettsäureaminoester wie z.B. Kokosfettsäureaminoethanolaminester oder -diethanolaminester in Betracht. Die Quaternierung wird vorzugsweise nicht Gegenwart von Wasser oder organischen Lösungsmitteln, sondern Dispergatoren bzw. Emulgatoren durchgeführt. Hierfür kommen beispielsweise folgende **Polyole** in Betracht:
- Glycerin,
- Alkylenglycole wie beispielsweise Ethylenglycol, Diethylenglycol, Propylenglycol;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche, mit 1 bis 8 Kohlenstoffen im Alkylrest wie beispielsweise Methyl- und Butylglucosid;
- Zuckeralkohole mit 5 bis 12 Kohlenstoffatomen wie beispielsweise Sorbit oder Mannit,
- Zucker mit 5 bis 12 Kohlenstoffatomen wie beispielsweise Glucose oder Saccharose;
- Aminozucker wie beispielsweise Glucamin.

Eine weitere Gruppe geeigneter Emulgatoren stellen Fettalkohole dar. Typische Beispiele hierfür sind Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol und Erucylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind technische Fettalkohole mit 12 bis 18 Kohlenstoffatomen wie beispielsweise Kokos-, Palm-, Palmkem- oder Talgfettalkohol. Weiterhin kommen als Emulgatoren auch die eingangs bereits genannten nichtionischen Tenside, vorzugsweise Alkyloligoglucoside oder ethoxylierte Fettsäurepartialglyceride in Frage. Im Rahmen des Herstellverfahrens können die quartären Glycerinstickstoffverbindungen die Dispergatoren bzw. Emulgatoren in solchen Mengen enthalten, daß ihr Anteil 10 bis 90, vorzugsweise 30 bis 70 Gew.-% - bezogen auf das Endprodukt - beträgt. Die Alkylierung der Glycerinstickstoffverbindungen kann in an sich bekannter Weise durchgeführt werden. Hierzu wird der Ester vorgelegt und mit dem Alkylierungsmittel - das man üblicherweise in äquimolaren Mengen oder leichtem Unterschuß einsetzt - bei erhöhten Temperaturen gerührt. Nach Abschluß der Reaktion kann nichtumgesetztes Alkylierungsmittel durch Zugabe einer geringen Menge Aminosäure, vorzugsweise Glycin, zerstört werden. Als Alkylierungsmittel kommen in diesem Zusammenhang Alkylhalogenide, oder Dialkylsulfate, vorzugsweise Methylchlorid oder Dimethylsulfat in Frage.

### Gewerbliche Anwendbarkeit

Die im Sinne der Erfindung einzusetzenden quartären Glycerinstickstoffverbindungen verleihen Haaren einen angenehmen Weichgriff, setzen die statische Aufladung zwischen den Keratinfasern herab und verbessern somit die Kämmbarkeit und lassen sich leicht in Wasser dispergieren. Des weiteren weisen sie auch in Form ihrer 90 Gew.-%igen Konzentrate eine vorteilhafte Viskosität auf, die auch bei längerer Lagerung weitgehend erhalten bleibt. Ein weiterer Gegenstand der Erfindung betrifft daher ihre Verwendung zur Herstellung von Haarbehandlungsmitteln, als da sind: Haarshampoos, Haarkuren, Haarspülungen und dergleichen, in denen sie in Mengen von 1 bis 50, vorzugsweise 2 bis 15 Gew.-% - bezogen auf die Mittel - enthalten sein können.

### Haarbehandlungsmittel

Die Haarbehandlungsmittel können als weitere Hilfs- und Zusatzstoffe Emulgatoren, Überfettungsmittel, Verdickungsmittel, Kationpolymere, Siliconverbindungen biogene Wirkstoffe, Filmbildner, Konservierungsmittel, Farb- und Duftstoffe enthalten. Als **Emulgatoren** kommen sowohl bekannte W/O- als auch O/W-Emulgatoren wie beispielsweise gehärtetes und ethoxyliertes Ricinusöl, Polyglycerinfettsäureester oder Polyglycerinpolyricinoleate in Frage. Als **Überfettungsmittel** können Substanzen wie beispielsweise polyethoxylierte Lanolinderivate, Lecithinderivate, Polyolfettsäureester, Monoglyceride und Fettsäurealkanolamide verwendet werden, wobei die letzteren gleichzeitig als Schaumstabilisatoren dienen. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon, Tenside wie beispielsweise Fettalkoholethoxylate mit eingeengter Homologenverteilung oder Alkyloligoglucoside sowie Elektrolyte wie Kochsalz und Ammoniumchlorid. Geeignete kationische Polymere sind beispielsweise kationischen Cellulosederivate, kationischen Stärke, Copolymere von Diallylammoniumsalzen und Acrylamiden, quatemierte Vinylpyrrolidon-Vinylimidazol-Polymere wie z.B. LUVIQUAT® (BASF AG, Ludwidshaften/FRG), Kondensationsprodukte von Polyglycolen und Aminen, quatemierte Kollagenpolypeptide wie beispielsweise Lauryldimonium hydroxypropyl hydrolyzed collagen (Lamequat®L, Grünau GmbH), Polyethylenimin, kationische Siliconpolymere wie z.B. Amidomethicone oder DOW CORNING 929, Dow Corning Co./US, Copolymere der Adipinsäure und Dimethylaminohydroxypropyldiethylentrimamin (CARTARETINE®, Sandoz/CH), Polyaminopolyamide wie z.B. beschrieben in der **FR-A 2252840** sowie deren vernetzte wasserlöslichen Polymere, kationische Chitinderivate wie beispielsweise quaterniertes Chitosan, gegebenenfalls mikrokristallin verteilt, kationischer Guar-Gum wie z.B. JAGUAR® C-B-S, JAGUAR® C-17, JAGUAR® C-16 der Celanese/US, quatemierte Ammoniumsalz-Polymere wie z.B. MIRAPOL® A-15, MIRAPOL® AD-1, MIRAPOL® AZ-1 der Miranol/US. Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor- und/oder alkylmodifizierte Siliconverbindungen. Unter **biogenen Wirkstoffen** sind beispielsweise Pflanzenextrakte und Vitaminkomplexe zu verstehen. Gebräuchliche **Filmbildner** sind beispielsweise Chitosan, mikrokristallines Chitosan, quatemiertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen. Als **Konservierungsmittel** eignen sich beispielsweise Phenoxyethanol, Formaldehydlösung, Parabene, Pentandiol oder Sorbinsäure. Als **Perlglanzmittel** kommen beispielsweise Glycoldistearinsäureester wie Ethylenglycoldistearat, aber auch Fettsäuremonoglycolester in Betracht. Als Farbstoffe können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, veröffentlicht im Verlag Chemie, Weinheim**, **1984, S.81-106** zusammengestellt sind. Diese Farbstoffe werden üblicherweise in Konzentrationen von 0,001 bis 0,1 Gew.-%, bezogen auf die gesamte Mischung, eingesetzt. Der Gesamtanteil der Hilfs- und Zusatzstoffe kann 1 bis 50, vorzugsweise 5 bis 40 Gew.-% - bezogen auf die Mittel - betragen.

### Beispiele

### I. Eingesetzte Tenside

(A1) **Armosoft PEQ1 :** Glycerin wurde mit 2 Mol Natriumchloracetat kondensiert, der Diester mit einem N-Methylethanolaminester auf Basis teilgehärteter Talgfettsäure kondensiert und anschließend mit Methylchlorid quaterniert.
(A2) **Armosoft PEQ2 :** Glycerin wurde mit 2 Mol Natriumchloracetat kondensiert, der Diester mit einem N-Methylethanolaminester auf Basis gehärteter Talgfettsäure kondensiert und anschließend mit Methylchlorid quaterniert.
(A3) **Armosoft PEQ3** : Glycerin wurde mit 2 Mol Natriumchloracetat kondensiert, der Diester mit einem N-Methylethanolaminester auf Basis Rapsfettsäure kondensiert und anschließend mit Methylchlorid quaterniert.
(A4) **Armosoft PEQ4** : Technisches Talgfettsäuremonoglycerid wurde mit 1 Mol Natriumchloracetat kondensiert, der Diester mit einem N-Methylethanolaminester auf Basis gehärteter Talgfettsäure kondensiert und anschließend mit Methylchlorid quaterniert.

Bei den oben genannten Armosoft-Typen A1 bis A4 handelt es sich um Produkte der Akzo, Düren/DE, die in der Europäischen Patentanmeldung EP-A 0639638 beschrieben werden.

### II. Herstellbeispiel A5:

In einem 500-ml-Dreihalskolben mit Rührer, Tropftrichter und Rückflußkühler wurde eine Mischung von 60 g (0,1 mol) eines Umsetzungsproduktes eines Kondensationsproduktes eines Esters aus einem technischem Laurinsäuremonoglycerid (Monomuls® 90 L-12) und Natriumchloracetat mit Kokosfettsäureaminoethylethanolaminester vorgelegt und bei etwa 50°C unter Rühren portionsweise mit 18 g - entsprechend 30 Gew.-% - C_{16/18}- Talgfettalkohol versetzt. Danach wurden innerhalb von 2 h 12 g (0,095 mol) Dimethylsulfat zugetropft. Nach Beendigung der Zugabe wurde die Mischung weitere 2 h bei 60°C gerührt und nichtumgesetztes DMS durch Zusatz von 0,4 g (0,005 mol) Gemisch wurde in praktisch quantitativer Ausbeute als hellfarbige, wachsartige Masse erhalten, die anschließend mechanisch geschuppt wurde.

### III. Viskositätsmessungen

Die quatemierten Glycerinstickstoffverbindungen A1 bis A5 sowie ein technisches Vergleichsprodukt B1 (Methylquaternierter Ditalgfettsäuretriethanolaminester-Methylsulfat-Salz, Dehyquart® AU-46, Henkel Ibérica S.A., Barcelona/ES) wurden in eine saure Haarspülung bestehend aus 6 Gew.-% Kationtensid und 1 Gew.-% Emulgator Cetylstearylalkohol-20 EO (Eumulgin® B2, Henkel KGaA, Düsseldorf/FRG) eingearbeitet (Wasser ad 100 Gew.-%). Der pH-Wert der Spülungen wurde auf 3,5 eingestellt. Die Emulsionsbildung erfolgte unter schwachem Rühren bei Raumtemperatur. In allen Fällen wurden homogene, kosmetisch elegante Emulsionen erhalten. Die Viskosität der Emulsionen wurde nach 1 bis 3 d Lagerung bestimmt (Brookfield RVT, 20°C, 10 Upm). Die Ergebnisse sind in Tabelle 1 zusammengefaßt. Die Beispiele 1 bis 4 sind erfindungsgemäß, Beispiel V1 dient zum Vergleich.

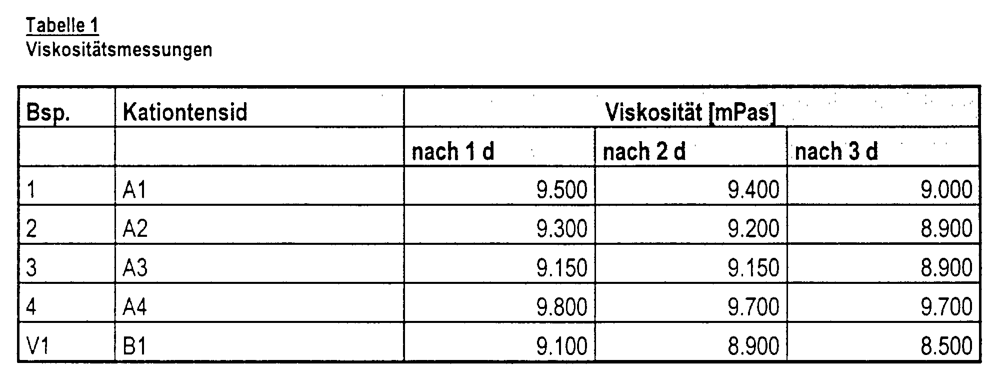

In den erfindungsgemäßen Beispielen 1 bis 4 zeigte sich, daß zur Emulsionsbildung eine im Vergleich deutlich geringere Scherleistung als im Fall des Vergleichsversuchs V1 erforderlich war. Im Hinblick auf die Viskosität der erfindungsgemäßen Beispiele 1 bis 4 wurde bei der Vergleichsemulsion V1 zwar ein ähnlicher Anfangswert erreicht, jedoch schon nach kurzer Lagerung eine rasche Viskositätsabnahme beobachtet.

### III. Avivage und antistatische Eigenschaften

Zur Bestimmung der Avivageeigenschaften wurden die quatemierten Glycerinstickstoffverbindungen A3 und A4, das Kationtensid B1 sowie Abmischungen von A4 mit den Tensiden C1 bis C4 im Gewichtsverhältnis 30:70 eingesetzt.
C1) Esterquat (Dehyquart® AU-56)
C2) Alkylpolyglucosid (Plantaren® APG-2000)
C3) Kokosfettsäure-N-methylglucamid
C4) Betain (Dehyton® PK)

Bei den genannten tensidischen Zusatzstoffen handelt es sich um Verkaufsprodukte der Henkel KGaA, Düsseldorf/DE. Die Meßmethoden werden nachfolgend beschrieben:

**Naßkämmbarkeit**. Die Naßkämmbarkeit wurde an braunem Haar (Alkinco #6634, Strähnenlänge 12 cm, Strähnenmasse 1 g) untersucht. Nach der Nullmessung wurden die Strähnen mit 100 ml der Formulierungen getränkt. Nach einer Einwirkzeit von 5 min wurden die Strähnen 1 min unter fließendem Wasser (1 l/min, 38°C) ausgespült. Die Strähnen wurden erneut vermessen und mit der Nullmessung verglichen. Der Fehler bei den Messungen betrug im Mittel 2 %, die statistische Sicherheit lag bei mindestens 99 %. Die Ergebnisse sind in Tabelle 2 dargestellt. Eine ausführliche Beschreibung der Meßmethoden befindet sich in **J.Soc.Cosm.Chem., 24, 782 (1973). Weichgriff**. Der Weichgriff des Haares wurde subjektiv von einer Gruppe aus 6 geschulten Personen bestimmt. Es werden die Mittelwerte aus Dreifachbestimmungen wiedergegeben. Es gelten die Noten 1 = sehr weich bis 4 = hart. Die Ergebnisse sind ebenfalls in Tabelle 2 zusammengefaßt.

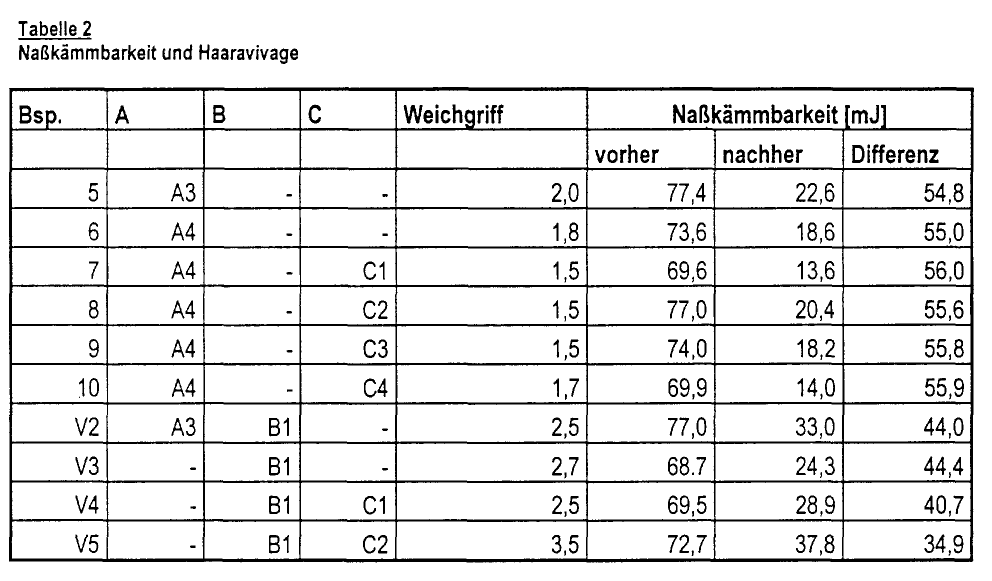

## Patentansprüche

1. Verwendung von quartären Glycerinstickstoffverbindungen der Formel **(I)**, 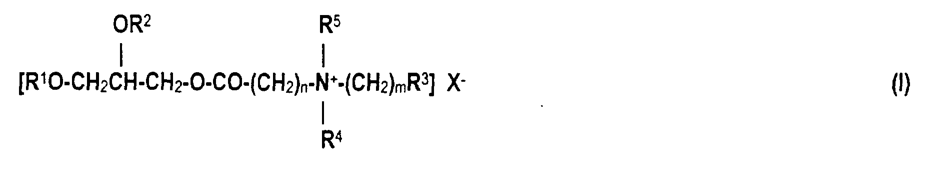 in der R¹ und R² unabhängig voneinander für Wasserstoff oder einen linearen oder verzweigten, gesättigten Acylrest mit 12 bis 22 Kohlenstoffatomen, R³ für eine Methylgruppe oder einen linearen oder verzweigten, gesättigten Oxyacylrest mit 12 bis 22 Kohlenstoffatomen, R⁴ und R⁵ unabhängig voneinander für einen gegebenenfalls hydroxysubstituierten Alkylrest mit 1 bis 4 Kohlenstoffatomen, n für Zahlen von 1 bis 3, m für 0 oder Zahlen von 1 bis 18 und X für Halogen oder Alkylsulfat steht, zur Herstellung von Haarbehandlungsmitteln.

2. Verwendung nach Anspruch 1, **dadurch gekennzeichnet,** daß man weitere kationische, nichtionische und/oder amphotere bzw. zwitterionische Tenside einsetzt.

3. Verwendung nach den Ansprüchen 1 und 2, **dadurch gekennzeichnet,** daß man weitere Tenside ein setzt, die ausgewählt sind aus der Gruppe, die von Esterquats, Alkyloligoglucosiden, Fettsäure-N-alkylglucamiden und Betainen gebildet wird.

4. Verwendung nach den Ansprüchen 1 bis 3, **dadurch gekennzeichnet,** daß man die quartären Glycerinstickstoffverbindungen der Formel **(I)** und die weiteren Tenside im Gewichtsverhältnis 10 : 90 bis 90:10 einsetzt.

## Claims

1. The use of quaternary glycerol nitrogen compounds corresponding to formula **(I)**: 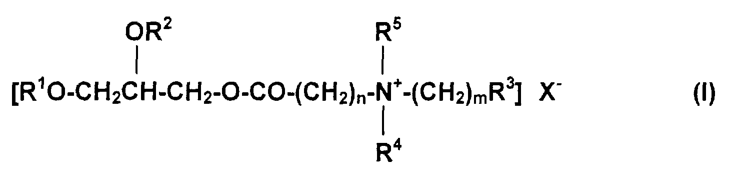 in which R¹ and R2 independently of one another represent hydrogen or a linear or branched, saturated acyl group containing 12 to 22 carbon atoms, R³ is a methyl group or a linear or branched, saturated oxyacyl group containing 12 to 22 carbon atoms, R⁴ and R⁵ independently of one another represent an optionally hydroxysubstituted alkyl group containing 1 to 4 carbon atoms, n is a number of 1 to 3, m is 0 or a number of 1 to 18 and X represents halogen or alkyl sulfate,
for the production of hair treatment preparations.

2. The use claimed in claim 1, characterized in that other cationic, nonionic and/or amphoteric or zwitterionic surfactants are used.

3. The use claimed in claims 1 and 2, characterized in that other surfactants selected from the group consisting of esterquats, alkyl oligoglucosides, fatty acid-N-alkyl glucamides and betaines are used.

4. The use claimed in claims 1 to 3, characterized in that the quaternary glycerol nitrogen compounds corresponding to formula (I) and the other surfactants are used in a ratio by weight of 10:90 to 90:10.

## Revendications

1. Utilisation de composés azotés du glycérol de formule (I) 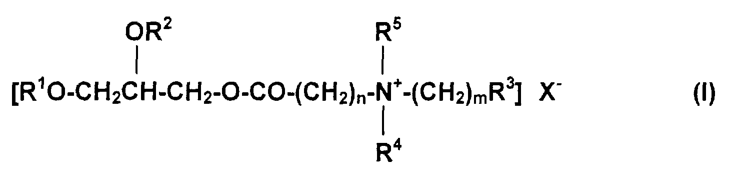 dans laquelle R¹ et R² indépendamment l'un de l'autre, représentent de l'hydrogène ou un reste acyle linéaire ou ramifié, saturé, ayant de 12 à 22 atomes de carbone, R³ représente un groupe méthyle ou un reste oxyacyle linéaire ou ramifié, saturé, ayant de 12 à 22 atomes de carbone, R⁴ et R⁵ indépendamment l'un de l'autre, représentent un reste alkyle éventuellement substitué par un hydroxy, ayant de 1 à 4 atomes de carbone, n représente des nombres allant de 1 à 3, m représente 0 ou des nombres allant de 1 à 18 et X, représente un halogène ou un alkylsulfate, en vue de la production d'agents de soins capillaires.

2. Utilisation selon la revendication 1,
caractérlsée en ce qu'
on utilise d'autres agents tensioactifs cationiques, non ioniques et/ou amphotères ou zwitterioniques.

3. Utilisation selon l'une quelconque des revendications 1 et 2,
caractérisée en ce qu'
on utilise d'autres agents tensioactifs qui sont choisis dans le groupe qui est formé des esterquats des alkyloligoglucosides, des N-alkylglucamides d'acide gras et des bétaïnes.

4. Utilisation selon l'une quelconque des revendications 1 à 3,
caractérisée en ce qu'
on met en oeuvre les composés azotés du glycérol quaternalres de formule (I) et les autres agents tensioactifs dans un rapport en poids de 10 : 90 à 90 : 10.
